# EUROPEAN PATENT SPECIFICATION

(11) **EP 3 797 674 B1**
(45) Date of publication and mention of the grant of the patent: **20.05.2026**
(21) Application number: 20199100.7
(22) Date of filing: 29.09.2020
(51) Int. Cl.: A61B 1/015, F04B 43/12, F04B 53/16

(54) **LIQUID SUPPLY DEVICE FOR ENDOSCOPE, LIQUID SUPPLY TUBE, AND LIQUID SUPPLY DEVICE BODY**
FLÜSSIGKEITSVERSORGUNGSVORRICHTUNG FÜR ENDOSKOP, FLÜSSIGKEITSVERSORGUNGSSCHLAUCH UND FLÜSSIGKEITSVERSORGUNGSVORRICHTUNGSKÖRPER
DISPOSITIF D'ALIMENTATION EN LIQUIDE POUR ENDOSCOPE, TUBE D'ALIMENTATION EN LIQUIDE ET CORPS DU DISPOSITIF D'ALIMENTATION EN LIQUIDE

(30) Priority: 30.09.2019 JP 2019179131
(43) Date of publication of application: 31.03.2021
(73) Proprietor: FUJIFILM Corporation, Tokyo 106-8620 (JP)
(72) Inventor: FUJIHIRA, Takuro, Japan, Kanagawa (JP); SATO, Jun, Japan, Kanagawa (JP)
(74) Representative: Dehns Germany Partnerschaft mbB

(56) References cited:
- EP-A1- 2 397 695
- WO-A1-2019/106185
- US-A1- 2013 315 763
- US-A1- 2019 030 235

## Description

### BACKGROUND OF THE INVENTION

### 1. Field of the Invention

The present invention relates to a liquid supply device for an endoscope, a liquid supply tube, and a liquid supply device body, and more particularly, to a liquid supply device for an endoscope, a liquid supply tube, and a liquid supply device body that are used to send liquid to a liquid supply pipe line provided in an endoscope or a treatment tool.

### 2. Description of the Related Art

In the field of an endoscope in the related art, liquid is supplied into a body to be observed using a liquid supply pipe line, such as a treatment tool-insertion channel or a water jet pipe inserted into an endoscope. For example, JP2006-325814A discloses a liquid supply device for an endoscope that sends liquid to the liquid supply pipe line. The liquid supply device for an endoscope sends liquid to the liquid supply pipe line by rotating a rotor and squeezing a part of the liquid supply tube with rollers arranged on the outer peripheral portion of the rotor in a state where the liquid supply tube is wound on the outer peripheral portion of the rotor. WO 2019/106185 discloses a peristaltic pump cassette. EP2397695 discloses a tube pump system.

### SUMMARY OF THE INVENTION

Meanwhile, since a liquid supply tube used in the liquid supply device for an endoscope sends a chemical solution or a wash solution to the liquid supply pipe line, the liquid supply tube is detached from the liquid supply device for an endoscope whenever being used. Then, the washed liquid supply tube or a new liquid supply tube is mounted on the liquid supply device for an endoscope.

Further, in a case where a liquid supply pipe line for a liquid supply destination is changed, the liquid supply tube may be interchanged with a liquid supply tube corresponding to the changed liquid supply pipe line among a plurality of different types of liquid supply tubes and the liquid supply tube may be then used.

However, the interchange of the liquid supply tube is not considered in the liquid supply device for an endoscope disclosed in JP2006-325814A at all. Accordingly, whenever a liquid supply tube is attached to the liquid supply device for an endoscope, the length and position of a winding portion of the liquid supply tube should be adjusted to an appropriate length and position. For this reason, there is a problem that labor is required for work for adjusting the length and position of the winding portion.

Accordingly, there is a problem that a liquid supply tube cannot be easily attached to and detached from the liquid supply device for an endoscope disclosed in JP2006-325814A.

The invention has been made in consideration with the above-mentioned circumstances, and an object of the invention is to provide a liquid supply device for an endoscope which a liquid supply tube can be easily attached to and detached from, the liquid supply tube, and a liquid supply device body.

In order to achieve the object of the invention, a liquid supply device for an endoscope comprises a liquid supply device body that sends liquid by rotating a rotor, which includes a plurality of rollers provided on an outer peripheral portion thereof, and squeezing a liquid supply tube with the rollers in a state where the liquid supply tube is wound around the rotor; the liquid supply tube includes a winding portion that is to be wound around the rotor, a first stopper part that is provided to be closer to a liquid destination side than the winding portion and protrudes in a radial direction of the tube orthogonal to an axial direction of the liquid supply tube, and a second stopper part that is provided to be closer to a liquid source side than the winding portion and protrudes in the radial direction of the tube; and the liquid supply device body includes a first restriction portion that is engaged with the first stopper part and restricts a position of the first stopper part, and a second restriction portion that is engaged with the second stopper part and restricts a position of the second stopper part.

It is preferable that the liquid supply device body includes a housing case defining a rotor housing chamber capable of housing the rotor and the housing case includes the first restriction portion and the second restriction portion.

It is preferable that the housing case includes a cover capable of opening and closing the rotor housing chamber, the cover includes a pressing piece protruding toward any one restriction portion of the first restriction portion or the second restriction portion, and the pressing piece presses the first stopper part or the second stopper part restricted by the one restriction portion and restricts movement of the first stopper part or the second stopper part in the radial direction of the tube.

It is preferable that the liquid supply tube includes a first joint to be connected to a first tube corresponding to the liquid destination side and the first joint includes the first stopper part.

It is preferable that the first tube includes a first connecting part provided at an end portion thereof opposite to the first joint and capable of being connected to a liquid supply pipe line provided in an endoscope or a treatment tool.

It is preferable that the liquid supply tube includes a second joint to be connected to a second tube corresponding to the liquid source side and the second joint includes the second stopper part.

It is preferable that the second tube includes a second connecting part provided at an end portion thereof opposite to the second joint and capable of being connected to a tank in which liquid is stored.

It is preferable that the liquid supply tube includes a first stopper-forming member to be integrally connected to the liquid supply tube and the first stopper part is formed at the first stopper-forming member.

It is preferable that the liquid supply tube includes a second stopper-forming member to be integrally connected to the liquid supply tube and the second stopper part is formed at the second stopper-forming member.

In order to achieve the object of the invention, a liquid supply tube according to an aspect of the invention is provided as claimed in claim 1.

It is preferable that the liquid supply tube according to this aspect of the invention further comprises a first joint to be connected to a first tube corresponding to the liquid destination side and the first joint includes the first stopper part.

In this aspect of the invention, it is preferable that the first tube includes a first connecting part provided at an end portion thereof opposite to the first joint and capable of being connected to a liquid supply pipe line provided in an endoscope or a treatment tool.

In this aspect of the invention, it is preferable that the liquid supply tube includes a second joint to be connected to a second tube corresponding to the liquid source side and the second joint includes the second stopper part.

In this aspect of the invention, it is preferable that the second tube includes a second connecting part provided at an end portion thereof opposite to the second joint and capable of being connected to a tank in which liquid is stored.

In this aspect of the invention, it is preferable that the liquid supply tube includes a first stopper-forming member to be integrally connected to the liquid supply tube and the first stopper part is formed at the first stopper-forming member.

In this aspect of the invention, it is preferable that the liquid supply tube includes a second stopper-forming member to be integrally connected to the liquid supply tube and the second stopper part is formed at the second stopper-forming member.

In order to achieve the object of the invention, a liquid supply device body according to another aspect of the invention is provided as claimed in claim 8.

It is preferable that the liquid supply device body according to this aspect of the invention further comprises a housing case defining a rotor housing chamber capable of housing the rotor and the housing case includes the first restriction portion and the second restriction portion.

In this aspect of the invention, it is preferable that the housing case includes a cover capable of opening and closing the rotor housing chamber, the cover includes a pressing piece protruding toward any one restriction portion of the first restriction portion or the second restriction portion, and the pressing piece presses the first stopper part or the second stopper part restricted by the one restriction portion and restricts movement of the first stopper part or the second stopper part in a radial direction of the tube orthogonal to the axial direction of the liquid supply tube.

According to the invention, a liquid supply tube can be easily attached and detached.

### BRIEF DESCRIPTION OF THE DRAWINGS

Fig. 1 is a diagram showing the entire configuration in which a liquid supply device for an endoscope according to an embodiment is shown together with an endoscope.
Fig. 2 is a diagram showing tube units that are connected to the endoscope and a treatment tool.
Fig. 3 is a diagram showing the configuration of an endoscope system comprising the endoscope.
Fig. 4 is a perspective view of the entire liquid supply device body.
Fig. 5 is an enlarged view of main portions of the liquid supply device body of which a pump head cover is open.
Fig. 6 is a perspective view showing the configuration of the tube unit.
Fig. 7 is a perspective view of which main portions are enlarged and which shows the shapes of restriction portions. shapes of restriction portions.
Fig. 8 is a diagram showing the configuration of identifiers that are used to identify the types of liquid supply tubes.
Fig. 9 is a diagram showing that a liquid supply tube is attached to the liquid supply device body.
Fig. 10 is a control block diagram relating to the control of the amount of liquid to be supplied by the liquid supply device body.
Fig. 11 is a diagram showing the configuration of a tube unit of another embodiment.
Fig. 12 is an exploded perspective view of the tube unit shown in Fig. 11.
Fig. 13 is a diagram showing that stopper parts of the tube unit shown in Fig. 11 are engaged with restriction portions.

### DESCRIPTION OF THE PREFERRED EMBODIMENTS

A liquid supply device for an endoscope, a liquid supply tube, and a liquid supply device body according to embodiments of the invention will be described below with reference to the accompanying drawings.

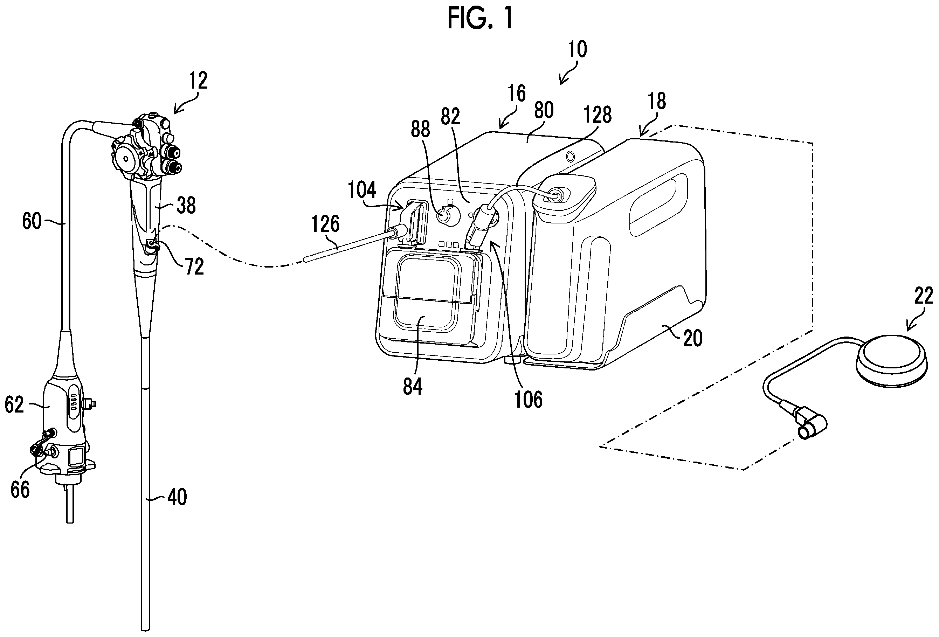

Fig. 1 is a diagram showing the entire configuration in which a liquid supply device 10 for an endoscope according to an embodiment is shown together with an endoscope 12.

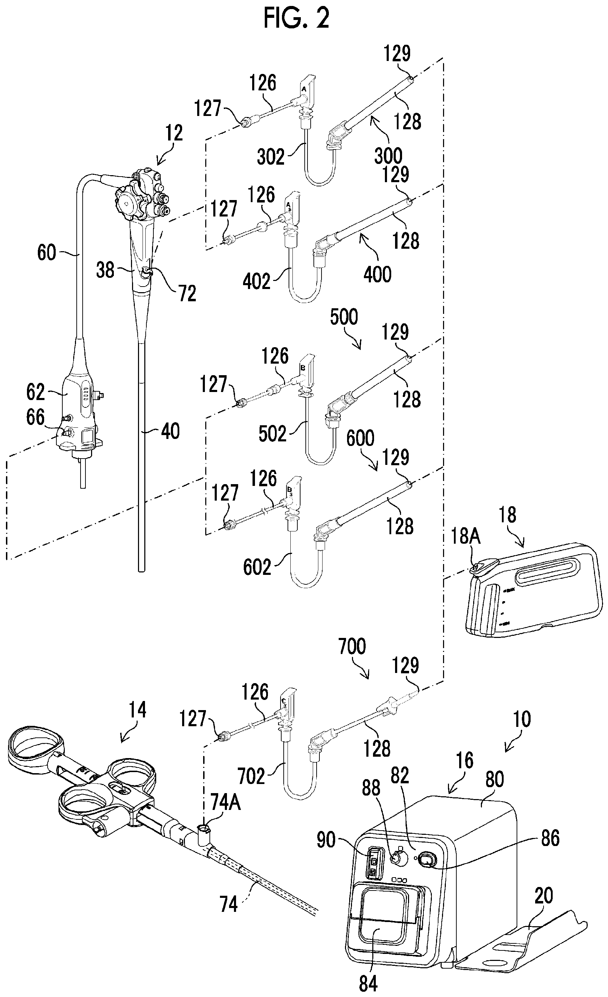

Fig. 2 is a diagram showing four different types of tube units 300, 400, 500, and 600 that are connected to a liquid supply pipe line of the endoscope 12 and a tube unit 700 that is connected to a liquid supply pipe line of a high-frequency knife 14 that is a treatment tool. These tube units 300 to 700 are attached to a liquid supply device body 16 forming the liquid supply device 10 for an endoscope so as to be interchangeable depending on the purpose or the intended use.

Further, the liquid supply device 10 for an endoscope includes a tank 18 in which liquid, such as a normal saline solution or a chemical solution, is stored, and the tank 18 is used in a state where the tank 18 is attachably and detachably placed on a tank tray 20 provided on the liquid supply device body 16. The liquid supply device body 16 and the tube units 300 to 700 will be described later.

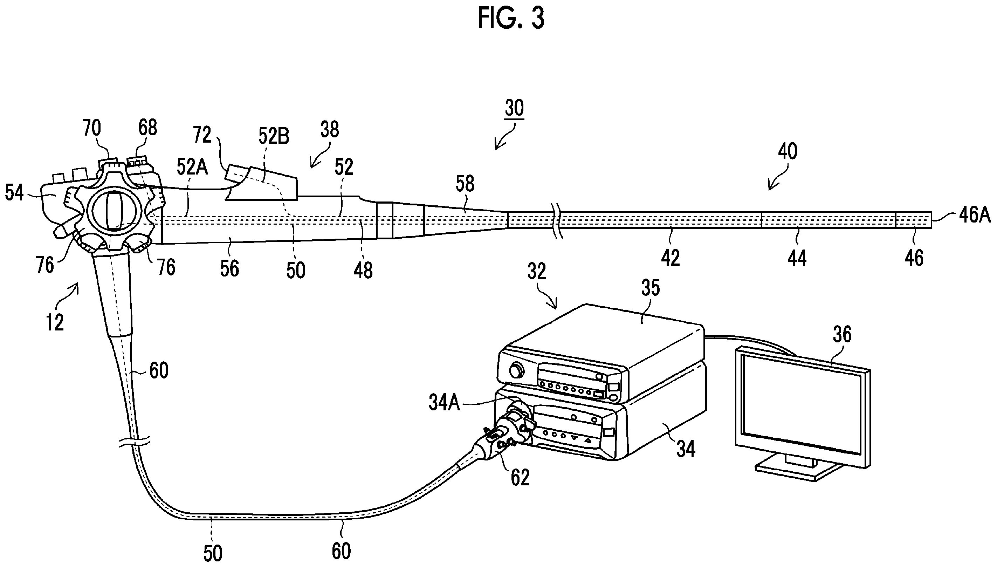

Next, an example of the endoscope 12 to be connected to the liquid supply device 10 for an endoscope will be described with reference to Fig. 3.

Fig. 3 is a diagram showing the configuration of an endoscope system 30 comprising the endoscope 12. The endoscope system 30 comprises the endoscope 12, a processor device 32 for an endoscope, and a display 36.

The endoscope 12 comprises an operation unit 38 and an insertion unit 40 that is provided on the distal end side of the operation unit 38 and is to be inserted into a body to be observed.

The insertion unit 40 includes a soft part 42, a bendable part 44, and a distal end part 46 that are arranged in this order toward a distal end from a proximal end. An air/water supply channel 48, a water jet pipe 50, and a treatment tool-insertion channel 52 shown in Fig. 3 by a dotted line, and the like are provided in the insertion unit 40. The air/water supply channel 48 is formed of a pipe line that jets liquid or air to an observation window (not shown) provided on a distal end face 46A of the distal end part 46 through a nozzle (not shown). The water jet pipe 50 is formed of a liquid supply pipe line that supplies liquid into the body to be observed, and the treatment tool-insertion channel 52 includes a pipe line into which a treatment tool is to be inserted and a liquid supply pipe line that supplies liquid into the body to be observed.

The operation unit 38 includes an operation unit body 54 and a grip part 56 connected to the operation unit body 54, and the proximal end portion of the insertion unit 40 is connected to the distal end side of the grip part 56 through a bending-proof pipe 58. The grip part 56 is a part that is gripped by an operator during an examination using the endoscope 12.

The operation unit body 54 is provided with a universal cable 60. A connector unit 62 is provided on the distal end side of the universal cable 60, and is connected to the processor device 32 for an endoscope. The processor device 32 for an endoscope comprises a light source device 34 and an image processing device 35. The light source device 34 is provided with a processor-side connector 34A to which the connector unit 62 is to be connected. Further, a display 36, which displays an image subjected to image processing by the image processing device 35, is connected to the image processing device 35. The endoscope system 30 is adapted to transmit electric power, optical signals, and the like between the endoscope 12 and the processor device 32 for an endoscope through a connector part, which includes the connector unit 62 and the processor-side connector 34A, in a non-contact manner. Accordingly, light generated from the light source device 34 is transmitted through an optical fiber cable (not shown), and is emitted from an illumination window (not shown) that is provided on the distal end face 46A of the distal end part 46. Furthermore, the optical signals of an image taken from the observation window are subjected to image processing by the image processing device 35 and are displayed on the display 36 as an image.

Here, as shown in Fig. 2, the connector unit 62 is provided with a liquid supply connector 66. A proximal end opening of the water jet pipe 50 (see Fig. 3) inserted into the insertion unit 40 and the universal cable 60 is connected to the liquid supply connector 66. A distal end opening of the water jet pipe 50 is disposed on the distal end face 46A of the distal end part 46 as a jet port. Two types of the tube units 500 and 600 are connected to the liquid supply connector 66 shown in Fig. 2 so as to be interchangeable.

Returning to Fig. 3, an air/water supply button 68 and a suction button 70 are installed side by side on the operation unit body 54. In a case where the air/water supply button 68 is operated by an operator, liquid or air is supplied to the air/water supply channel 48. Accordingly, liquid or air is jetted to the observation window. Further, in a case where the suction button 70 is operated by an operator, liquid present in the body to be observed is sucked from a distal end opening of the treatment tool-insertion channel 52, which is disposed on the distal end face 46A of the distal end part 46, through the treatment tool-insertion channel 52.

The treatment tool-insertion channel 52 branches into a suction passage 52A and a treatment tool-insertion passage 52B in the grip part 56. The suction passage 52A is connected to a suction port (not shown) of the connector unit 62 through a cylinder (not shown) of the suction button 70 and the universal cable 60, and the suction port is connected to a suction pump (not shown). Furthermore, the treatment tool-insertion passage 52B is connected to a forceps port 72 protruding from the grip part 56. Two types of the tube units 300 and 400 shown in Fig. 2 are connected to the forceps port 72 so as to be interchangeable.

Further, the high-frequency knife 14 shown in Fig. 2 is introduced into the treatment tool-insertion channel 52 from the forceps port 72 shown in Fig. 3 through the treatment tool-insertion passage 52B. The high-frequency knife 14 is provided with a liquid supply pipe line 74 shown in Fig. 2 by a dotted line, and the tube unit 700 is connected to a proximal end opening 74A of the liquid supply pipe line 74.

Returning to Fig. 3, the operation unit body 54 is provided with a pair of angle knobs 76 and 76 that is used to perform an operation for bending the bendable part 44. The pair of angle knobs 76 and 76 is coaxially provided so as to be rotationally movable.

The schematic configuration of the endoscope system 30 shown in Fig. 3 has been described above. The liquid supply device 10 for an endoscope shown in Figs. 1 and 2 will be described below.

As shown in Fig. 1, the liquid supply device 10 for an endoscope comprises the liquid supply device body 16, the tank 18, and a foot switch 22. Five different types of tube units 300 to 700 shown in Fig. 2 are attached to the liquid supply device body 16 so as to be interchangeable depending on the purpose or the intended use.

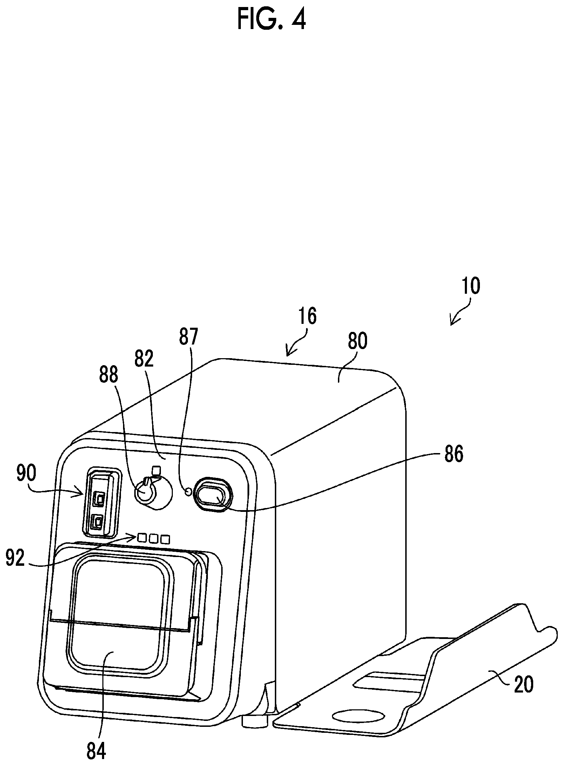

Fig. 4 is a perspective view of the entire liquid supply device body 16.

As shown in Fig. 4, the liquid supply device body 16 includes a housing 80. A pump head cover (hereinafter abbreviated as a "cover") 84, a power switch 86, a power switch indicator lamp 87, a flow rate adjustment knob 88, a liquid supply tube-detection unit 90, and a liquid supply tube-detection indicator lamp 92 are provided at predetermined positions on an operation panel 82 forming the front surface of the housing 80. The operation panel 82 is formed of a surface that is inclined obliquely upward.

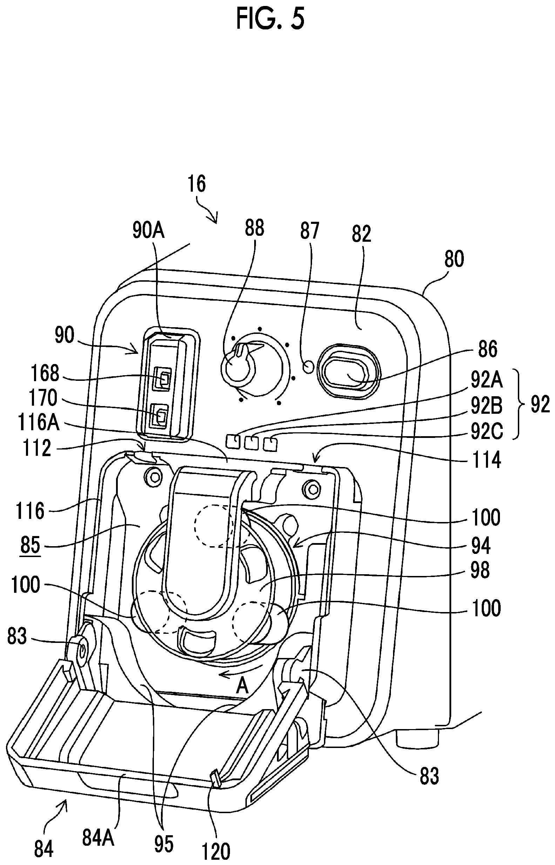

Fig. 5 is an enlarged view of main portions of the liquid supply device body 16 of which the cover 84 is open, and is a diagram showing that a pump 94 having been covered with the cover 84 is exposed to the outside.

As shown in Figs. 4 and 5, the cover 84 is formed in a rectangular shape as a whole and is provided on the liquid supply device body 16 so as to be openable and closable through hinge portions 83 and 83 that are provided on both left and right sides of a lower portion thereof.

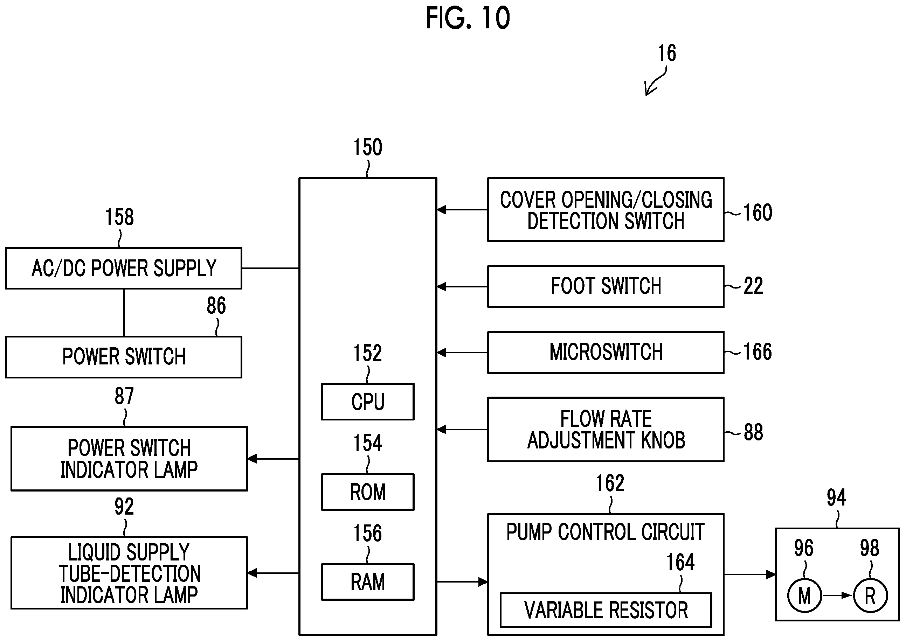

As shown in Fig. 5, the pump 94 is provided in a rotor housing chamber 85 that is exposed to the outside since the cover 84 is open. The pump 94 includes a DC brushless motor (see Fig. 10. Hereinafter abbreviated as a "motor") 96 that is installed in the housing 80, a disc-shaped rotor 98 that is rotated by the motor 96, and three rollers 100, 100, and 100 that are mounted on the outer peripheral portion of the rotor 98 at regular intervals. A liquid supply tube of one tube unit selected from the five types of tube units 300 to 700 is attached to the pump 94 provided in the liquid supply device body 16 in a state where the liquid supply tube is wound in a U shape on the outer peripheral portion of the rotor 98. The liquid supply tube will be described later.

According to the pump 94 having the above-mentioned configuration, a part of the liquid supply tube is squeezed by the roller 100 and the rotor 98 is rotated in a counterclockwise direction, which is indicated in Fig. 5 by an arrow A, by the motor 96. Accordingly, a position where the liquid supply tube is squeezed is moved in the counterclockwise direction by the rotor 98, so that liquid present in the liquid supply tube is sent to a part to which liquid is to be supplied. In this specification, the water jet pipe 50, the treatment tool-insertion channel 52, and the liquid supply pipe line 74 will be described as an example of the part to which liquid is to be supplied.

The tube unit 300 among the tube units 300 to 700 shown in Fig. 2 will be described below by way of example.

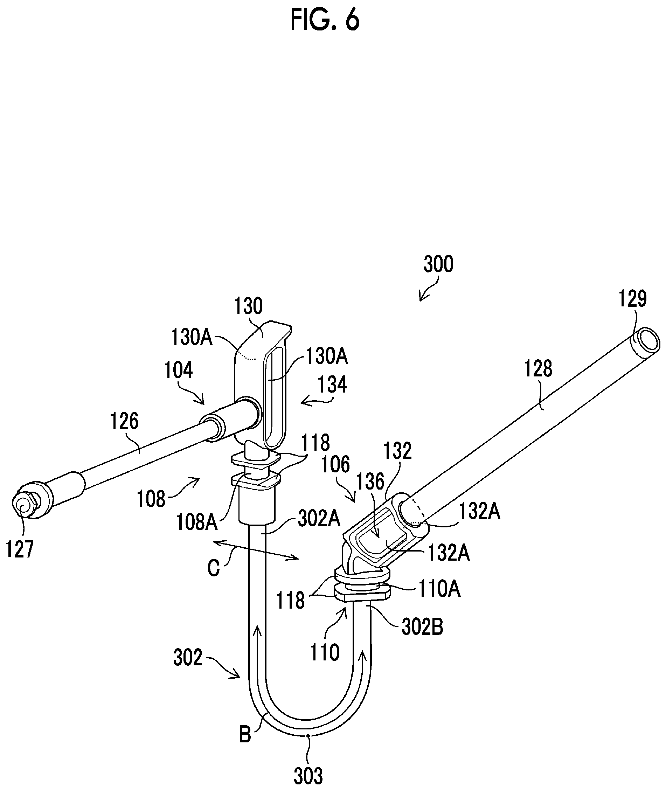

Fig. 6 is a perspective view showing the configuration of the tube unit 300. The same components of the tube units 400 to 700 shown in Fig. 2 as the components of the tube unit 300 shown in Fig. 6 will be denoted by the same reference numerals as those shown in Fig. 6 and the description thereof will be omitted.

The tube unit 300 includes a liquid supply tube 302. The liquid supply tube 302 is an elastic tube made of, for example, silicone rubber, and includes a winding portion 303 that is a portion to be wound on the rotor 98 in a case where the liquid supply tube 302 is to be wound on the rotor 98. Further, a joint 104 is provided at an end portion 302A that is closer to a liquid destination side than the winding portion 303, and a joint 106 is provided at an end portion 302B that is closer to a liquid source side than the winding portion 303. The joint 104 is an example of a first j oint and the joint 106 is an example of a second joint.

The joint 104 is provided with a stopper part 108 and the joint 106 is provided with a stopper part 110. Each of these stopper parts 108 and 110 is formed in the shape of a flange so as to protrude in a radial direction of the tube (the direction of an arrow C shown in Fig. 6) orthogonal to an axial direction of the liquid supply tube 302 (the direction of an arrow B shown in Fig. 6). Here, the stopper part 108 is an example of a first stopper part and the stopper part 110 is an example of a second stopper part.

As shown in Fig. 6, the stopper parts 108 and 110 include shaft portions 108A and 110A extending in the axial direction of the tube (the direction of the arrow B shown in Fig. 6), respectively. Further, the stopper part 108 includes a pair of stopper pieces 118 and 118 that is disposed to be spaced apart from each other in the axial direction of the tube (the direction of the arrow B shown in Fig. 6) with the shaft portion 108A interposed therebetween, and the stopper part 110 includes a pair of stopper pieces 118 and 118 that is disposed to be spaced apart from each other in the axial direction of the tube with the shaft portion 110A interposed therebetween.

As shown in Fig. 5, the liquid supply device body 16 is provided with a restriction portion 112 that is engaged with the stopper part 108 (see Fig. 6) and restricts the position of the stopper part 108 and a restriction portion 114 that is engaged with the stopper part 110 (see Fig. 6) and restricts the position of the stopper part 110. These restriction portions 112 and 114 are provided so as to be spaced apart from each other at an upper side portion 116A of a rectangular housing case 116 that is formed on the operation panel 82 and defines the rotor housing chamber 85. Here, the restriction portion 112 is an example of a first restriction portion and the restriction portion 114 is an example of a second restriction portion.

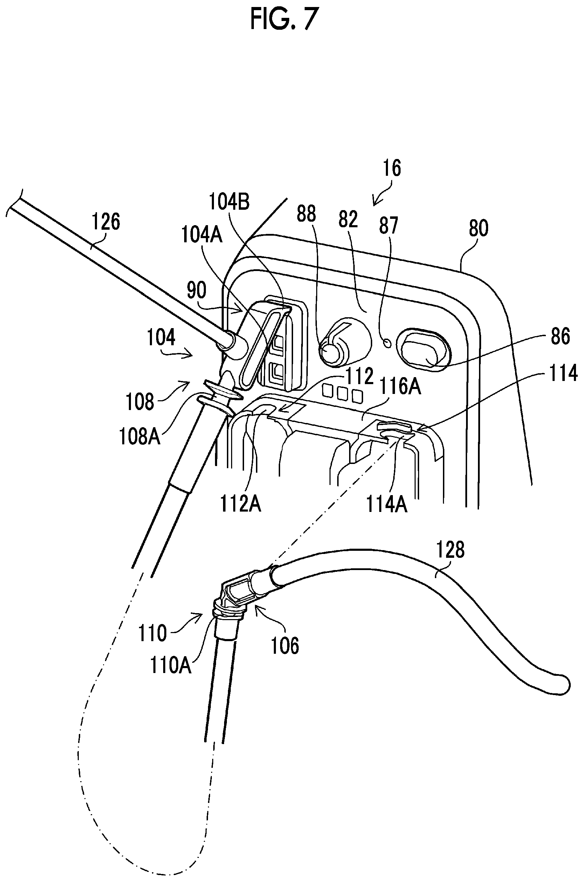

Further, the restriction portions 112 and 114 shown in Fig. 5 include grooves 112A and 114A (see Fig. 7) into which the shaft portions 108A and 110A are to be inserted, respectively. The grooves 112A and 114A are formed by the notching of a part of the upper side portion 116A.

Fig. 7 is a perspective view of which main portions are enlarged and which shows examples of the shapes of the restriction portions 112 and 114.

As shown in Fig. 7, the groove 112A of the restriction portion 112 is formed in a U shape and the groove 114A of the restriction portion 114 is formed in an L shape. Accordingly, the stopper part 108 is engaged with the restriction portion 112 by an operation for pushing the shaft portion 108A into the groove 112A. Further, the stopper part 110 is engaged with the restriction portion 114 by an operation for pushing the shaft portion 110A into the groove 114A in an L shape.

Since the pairs of stopper pieces 118 and 118 are engaged with the upper side portion 116A in this case, the movement of the stopper parts 108 and 110 to both sides in the axial direction of the tube (the direction of the arrow B shown in Fig. 6) is restricted. Since a shift in the position of the liquid supply tube 302 in the axial direction of the tube (the direction of the arrow B shown in Fig. 6) is prevented by this configuration during the rotation of the rotor 98, an operation for stably supplying liquid by the liquid supply device body 16 is achieved. In terms of the restriction of movement of the stopper parts, the pairs of stopper pieces 118 and 118 are provided on each of both the stopper parts 108 and 110.

Further, the diameters of the shaft portions 108A and 110A of the stopper parts 108 and 110 are different from each other, and the widths of the grooves 112A and 114A of the restriction portions 112 and 114 correspond to the diameters of the shaft portions 108A and 110A, respectively. Since the stopper parts 108 and 110 and the restriction portions 112 and 114 are formed as described above, the incorrect attachment of the stopper part 108 to the restriction portion 114 or the incorrect attachment of the stopper part 110 to the restriction portion 112 can be prevented.

According to the liquid supply device 10 for an endoscope of the embodiment, due to this configuration, it is possible to attach the liquid supply tube 302 to the liquid supply device body 16 merely by engaging the stopper part 108 with the restriction portion 112 and engaging the stopper part 110 with the restriction portion 114 in a state where the winding portion 303 is wound on the outer peripheral portion of the rotor 98. Further, it is possible to detach the liquid supply tube 302 from the liquid supply device body 16 merely by detaching the stopper part 108 from the restriction portion 112 and detaching the stopper part 110 from the restriction portion 114. Accordingly, each of the liquid supply tubes of the five types of tube units 300 to 700 can be simply attached to and detached from the liquid supply device body 16.

Furthermore, as shown in Fig. 5, an upper side portion 84A of the cover 84 includes a pressing piece 120 that has the shape of the tip of a sword and protrudes toward the restriction portion 114. In a case where the cover 84 is closed, the pressing piece 120 presses the shaft portion 110A of the stopper part 110 engaged with the groove 114A of the restriction portion 114. Accordingly, the movement of the stopper part 110 in the groove 114A in the radial direction of the tube (the direction of the arrow C shown in Fig. 6) is restricted by the pressing piece 120. Therefore, in a case where the cover 84 is closed, a state where the stopper part 110 is engaged with the restriction portion 114 is maintained. The pressing piece 120 may also be provided on a side corresponding to the restriction portion 112 without being limited to a side corresponding to the restriction portion 114. Accordingly, in a case where the cover 84 is closed, a state where the stopper part 108 is engaged with the restriction portion 112 is maintained.

Further, each of the joints 104 and 106 shown in Fig. 6 is formed of a coupler that connects tubes. A tube 126 corresponding to the liquid destination side is connected to the joint 104. The tube 126 includes a connecting pipe 127 provided at an end portion thereof opposite to the joint 104, and the connecting pipe 127 is attachably and detachably connected to the forceps port 72 shown in Fig. 2. The tube units 400 to 700 also include the same tubes 126, the tube 126 of the tube unit 400 is attachably and detachably connected to the forceps port 72 through the connecting pipe 127, the tube 126 of each of the tube unit 500 and 600 is attachably and detachably connected to the liquid supply connector 66 through the connecting pipe 127, and the tube 126 of the tube unit 700 is attachably and detachably connected to the proximal end opening 74A of the high-frequency knife 14 through the connecting pipe 127. Here, the tube 126 is an example of a first tube and the connecting pipe 127 is an example of a first connecting part.

Furthermore, a tube 128 corresponding to the liquid source side is connected to the joint 106 shown in Fig. 6. The tube 128 includes a distal end opening part 129 provided at an end portion thereof opposite to the joint 106, and the distal end opening part 129 is inserted into the tank 18 through a connection port 18A of the tank 18 shown in Fig. 2 and is positioned below the liquid level in the tank 18. The tube units 400 to 700 also include the same tubes 128, and the distal end opening part 129 of each of the tube 128 of the tube unit 400, the tubes 128 of the tube units 500 and 600, and the tube 128 of the tube unit 700 is inserted into the tank 18 through the connection port 18A of the tank 18 and is positioned below the liquid level in the tank 18. The tube 128 is an example of a second tube and the distal end opening part 129 is an example of a second connecting part.

As shown in Fig. 6, the joint 104 includes a joint body part 130 having a substantially rectangular parallelepiped shape and the joint 106 includes a joint body part 132 having a substantially rectangular parallelepiped shape. The joint body part 130 includes a knob portion 134 comprising a pair of knob surfaces 130A and 130A opposite to each other, and the joint body part 132 includes a knob portion 136 comprising a pair of knob surfaces 132A and 132A opposite to each other. Since the joints 104 and 106 include the knob portions 134 and 136, an operator can pick up the knob portions 134 and 136 with fingers and easily engage the stopper parts 108 and 110 with the restriction portions 112 and 114.

Here, the tube units 300 to 700 will be described with reference to Fig. 2.

As already described above, the tube units 300 and 400 are connected to the forceps port 72. The tube unit 300 is a reuse type of tube unit that is sterilized and washed and is reused on each examination, and the tube unit 400 is a disposable type of tube unit that is discarded after an examination. Further, the tube units 500 and 600 are connected to the liquid supply connector 66. The tube unit 500 is a reuse type of tube unit that is sterilized and washed and is reused on each examination, and the tube unit 600 is a disposable type of tube unit that is discarded after an examination. Furthermore, the tube unit 700 is connected to the proximal end opening 74A of the high-frequency knife 14, and is a disposable type of tube unit that is discarded after an examination.

Since the inner diameter of the treatment tool-insertion channel 52 shown in Fig. 3 is larger than the inner diameter of each of the water jet pipe 50 and the liquid supply pipe line 74, the amount (mL/min) of liquid supplied by the treatment tool-insertion channel 52 is set to be larger than the amount of liquid supplied by each of the water jet pipe 50 and the liquid supply pipe line 74. Accordingly, the inner diameters of the liquid supply tubes 302 and 402 are larger than the inner diameters of the liquid supply tubes 502, 602, and 702.

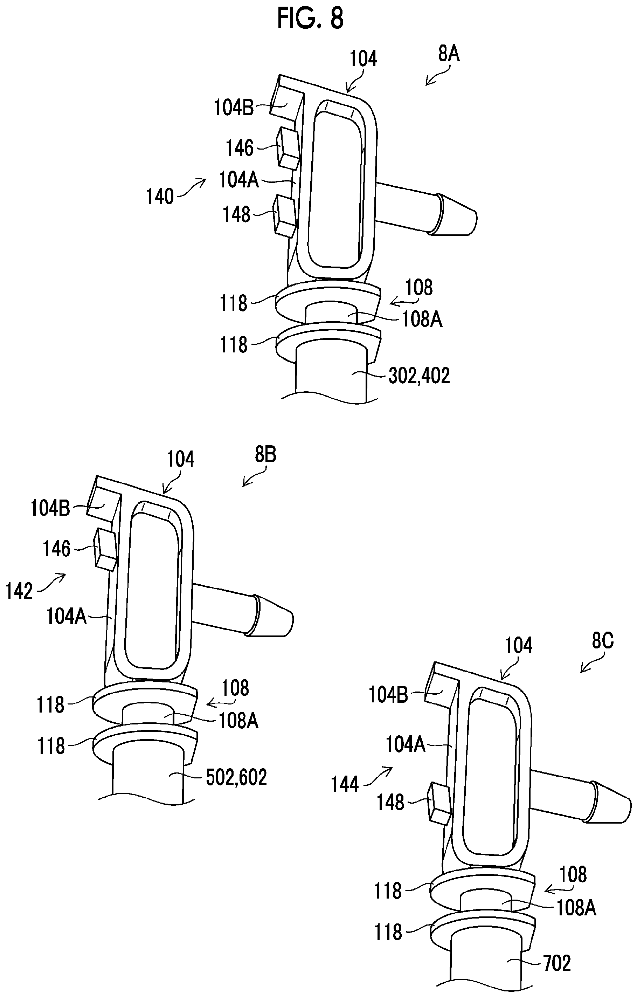

Next, examples of identifiers used to identify the types of the liquid supply tubes 302, 402, 502, 602, and 702 will be described with reference to Fig. 8.

These identifiers 140, 142, and 144 are formed to have different shapes by combinations of two projection portions 146 and 148. Here, the projection portions 146 and 148 are examples of mechanical identification information.

The identifier 140 is the identifier of each of the liquid supply tubes 302 and 402 where two projection portions 146 and 148 are provided on a rear surface 104A of the joint 104 so as to be spaced apart from each other in a vertical direction. Further, the identifier 142 is the identifier of each of the liquid supply tubes 502 and 602 where one projection portion 146 is provided at the upper portion of the rear surface 104A of the joint 104. Furthermore, the identifier 144 is the identifier of the liquid supply tube 702 where one projection portion 148 is provided at the lower portion of the rear surface 104A of the joint 104.

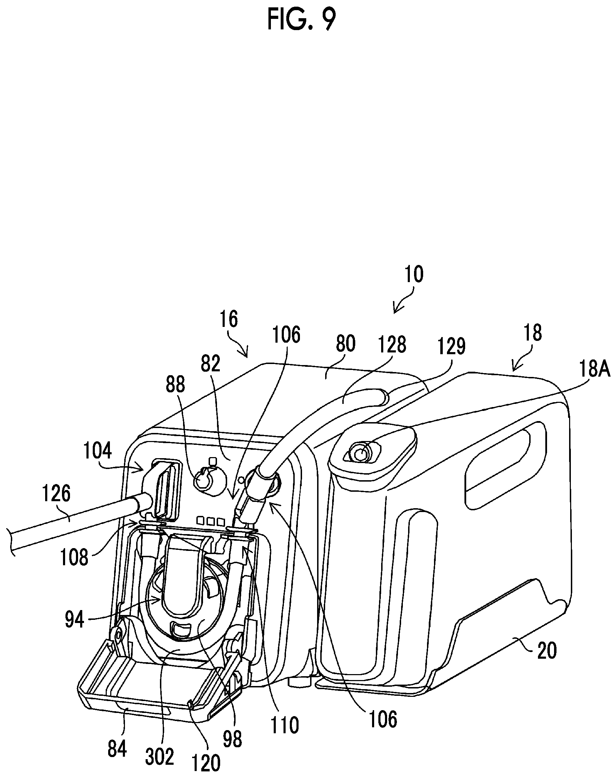

The identifier 140, 142, or 144 is provided at a position that can be recognized by the liquid supply tube-detection unit 90 in a case where the liquid supply tube 302 (402, 502, 602, or 702) is attached to the liquid supply device body 16 as shown in Fig. 9. Then, in a case where the joint 104 is connected to the liquid supply tube-detection unit 90, the identifier 140, 142, or 144 is recognized by an identifier recognition unit of the liquid supply device body 16. The identifier recognition unit will be described later.

A protruding portion 104B is formed at the upper portion of the rear surface 104A of the joint 104 as shown in Fig. 8 and a recessed portion 90A (see Fig. 5) is formed at the upper portion of the liquid supply tube-detection unit 90. Accordingly, in a case where the protruding portion 10B is engaged with the recessed portion 90A (see Fig. 7), the joint 104 is easily positioned with respect to the liquid supply tube-detection unit 90.

Fig. 10 is a control block diagram relating to the control of the amount of liquid to be supplied by the liquid supply device body 16.

As shown in Fig. 10, the amount of liquid to be supplied by the liquid supply device body 16 is controlled by a central processing unit (CPU) 152 provided in a control circuit 150. A plurality of circuits, such as a read only memory (ROM) 154 for storing programs and a random access memory (RAM) 156 in which data are temporarily stored, are provided in the control circuit 150.

Data (flow rate range-setting data) used to set the flow rate range of liquid for each type of a liquid supply tube according to a detection signal output from a microswitch 166 to be described later are stored in the ROM 154. Power is supplied to the respective circuits, which are provided in the control circuit 150, from an AC/DC power supply 158. Further, the microswitch 166 is an example of the identifier recognition unit.

A cover opening/closing detection switch 160 for detecting the opening/closing of the cover 84, the foot switch 22, the flow rate adjustment knob 88, and a pump control circuit 162 for causing the pump 94 to operate according to the operation position of the flow rate adjustment knob 88 are connected to the control circuit 150. The pump control circuit 162 includes a current switching circuit that switches current flowing in the motor 96, and a variable resistor 164 is used as an example of the current switching circuit. The control circuit 150 causes each circuit to operate in a case where a signal indicating the closing of the cover 84 is output from the cover opening/closing detection switch 160.

The control circuit 150 sets a flow rate, which is sent from the pump 94, in a flow rate range corresponding to the type of the liquid supply tube connected to the microswitch 166 by reading out data from the ROM 154 according to a detection signal output from the microswitch 166 and outputting the data to the pump control circuit 162. Further, the control circuit 150 adjusts a flow rate in the range of the flow rate range according to the operation position of the flow rate adjustment knob 88. Specifically, the flow rate adjustment knob 88 can be operated in an operation range of 0 to 100%. For example, in a case where the flow rate adjustment knob is in an operation position of 50%, the control circuit 150 sets the rotational speed of the rotor 98 so that liquid is supplied at a medium flow rate in the flow rate range. That is, the control circuit 150 sets the amount of liquid to be supplied to the amount of liquid to be supplied (a ratio to the flow rate range) that corresponds to the operation position of the flow rate adjustment knob 88 in the set flow rate range. Accordingly, the amount of liquid to be supplied (a ratio to the flow rate range) corresponding to the operation position of the flow rate adjustment knob 88 is achieved in the flow rate range that is set for each type of a liquid supply tube.

Here, the liquid supply tube-detection unit 90 shown in Fig. 5 includes the microswitch 166 (see Fig. 10) that can mechanically read the projection portions 146 and 148 (see Fig. 8).

The microswitch 166 includes two actuators 168 and 170 (see Fig. 5) that are pressed by the projection portions 146 and 148 (see Fig. 8) in a case where the joint 104 is connected to the liquid supply tube-detection unit 90 (see Fig. 5) (see Fig. 9), and two terminal parts (not shown) detecting that the actuators 168 and 170 are pressed and outputting a detection signal to the control circuit 150.

Specifically, for example, in a case where the joint 104 of the liquid supply tube 302 is connected to the liquid supply tube-detection unit 90, the actuator 168 is pressed by the projection portion 146 and the actuator 170 is pressed by the projection portion 148. Accordingly, a detection signal indicating that the actuators 168 and 170 are pressed is output to the control circuit 150 from the terminal parts.

The control circuit 150 recognizes the connection of the liquid supply tube 302 to the liquid supply device body 16 on the basis of the detection signal, and changes the operating condition of the pump 94. That is, in a case where the liquid supply tube 302 is connected, the range of the rotational speed of the rotor 98 is set so that the amount of liquid to be supplied is in the flow rate range of, for example, 400 mL/min to 700 mL/min. Then, the amount of liquid to be supplied is set to the amount of liquid to be supplied (a ratio to the flow rate range) that corresponds to the operation position of the flow rate adjustment knob 88 in the set flow rate range. Accordingly, in a case where the foot switch 22 is pressed, the rotor 98 is rotated at a rotational speed corresponding to the amount of liquid to be supplied (a ratio to the flow rate range) corresponding to the operation position of the flow rate adjustment knob 88 and liquid corresponding to the rotational speed is sent to the treatment tool-insertion channel 52.

The same applies to a case where the joint 104 of the liquid supply tube 402 is connected to the liquid supply tube-detection unit 90.

Since the actuator 168 is pressed by the projection portion 146 in a case where the joint 104 of the liquid supply tube 502 is connected, a detection signal indicating that the actuator 168 is pressed is output to the control circuit 150 from the terminal parts. Accordingly, the range of the rotational speed of the rotor 98 is set so that the amount of liquid to be supplied, which can be adjusted by the flow rate adjustment knob 88, is in the flow rate range of, for example, 60 mL/min to 190 mL/min. Then, the amount of liquid to be supplied is set to the amount of liquid to be supplied (a ratio to the flow rate range) that corresponds to the operation position of the flow rate adjustment knob 88 in the set flow rate range.

The same applies to a case where the joint 104 of the liquid supply tube 602 is connected to the liquid supply tube-detection unit 90.

Since the actuator 170 is pressed by the projection portion 148 in a case where the joint 104 of the liquid supply tube 702 is connected, a detection signal indicating that the actuator 170 is pressed is output to the control circuit 150 from the terminal parts. Accordingly, the range of the rotational speed of the rotor 98 is set so that the amount of liquid to be supplied, which can be adjusted by the flow rate adjustment knob 88, is in the flow rate range of, for example, 90 mL/min to 190 mL/min. Then, the amount of liquid to be supplied is set to the amount of liquid to be supplied (a ratio to the flow rate range) that corresponds to the operation position of the flow rate adjustment knob 88 in the set flow rate range.

Further, the control circuit 150 controls the turn-on of the liquid supply tube-detection indicator lamp 92 on the basis of a detection signal output from the microswitch 166 (that is, an identifier recognized by the microswitch 166 that is the identifier recognition unit). Specifically, in a case where the joint 104 of the liquid supply tube 302 or 402 is connected, the control circuit 150 turns on a lamp 92A (see Fig. 5) of the liquid supply tube-detection indicator lamp 92. Furthermore, in a case where the joint 104 of the liquid supply tube 502 or 602 is connected, the control circuit 150 turns on a lamp 92B (see Fig. 5) of the liquid supply tube-detection indicator lamp 92. Moreover, in a case where the joint 104 of the liquid supply tube 702 is connected, the control circuit 150 turns on a lamp 92C (see Fig. 5) of the liquid supply tube-detection indicator lamp 92. Accordingly, an operator can grasp one of the liquid supply tubes 302 to 702, which is connected to the liquid supply device body 16, merely by checking the turn-on of the lamp 92A, 92B, and 92C. The microswitch 166 and the control circuit 150 are an example of the identifier recognition unit. Further, the microswitch 166 is an example of a mechanical reading unit.

Next, the action of the liquid supply device 10 for an endoscope according to the embodiment will be described.

A case where the tube unit 300 is selected and used among the tube units 300 to 700 will be described.

First, as shown in Fig. 5, an operator opens the cover 84 of the liquid supply device body 16 to expose the rotor 98 to the outside. Next, the operator winds the winding portion 303 (see Fig. 6) of the liquid supply tube 302 in a U shape on the outer peripheral portion of the rotor 98. Then, as shown in Fig. 7, the operator engages the stopper part 108 with the restriction portion 112 to restrict the position of the stopper part 108 and engages the stopper part 110 with the restriction portion 114 to restrict the position of the stopper part 110.

After that, the operator connects the joint 104 of the tube unit 300 to the liquid supply tube-detection unit 90. Specifically, after engaging the protruding portion 104B of the joint 104 with the recessed portion 90A of the liquid supply tube-detection unit 90 to position the joint 104 on the liquid supply tube-detection unit 90 as shown in Figs. 5 and 7, the operator pushes the joint 104 to the liquid supply tube-detection unit 90 as shown in Fig. 9. Accordingly, the operator can easily and reliably connect the joint 104 to the liquid supply tube-detection unit 90.

Then, the operator closes the cover 84 as shown in Fig. 1. The operator can fix the tube unit 300 to the liquid supply device body 16 with the above-mentioned work.

In this case, since the pressing piece 120 shown in Fig. 5 presses the shaft portion 110A of the stopper part 110 to the groove 114A and restricts the movement of the stopper part 110 in the radial direction of the tube (the direction of the arrow C shown in Fig. 6), a shift in the position of the stopper part 110 with respect to the restriction portion 114 can be prevented. Work for connecting the joint 104 shown in Fig. 7 to the liquid supply tube-detection unit 90 may be performed before work for engaging the stopper part 108 with the restriction portion 112.

Further, it is preferable that the positions of the stopper parts 108 and 110 on the liquid supply tube 302 in the axial direction of the tube (the direction of the arrow B shown in Fig. 6) are set to positions where the liquid supply tube 302 is connected to the liquid supply device body 16 without receiving excessive stress (unnecessary tension) in the axial direction of the tube (the direction of the arrow B shown in Fig. 6). In a case where the stopper parts 108 and 110 are provided at these positions, the stopper part 108 is engaged with the restriction portion 112, and the stopper part 110 is engaged with the restriction portion 114, the length and position of the winding portion 303 of the liquid supply tube 302 can be set to an appropriate length and an appropriate position. Accordingly, since work for adjusting the length and position of the winding portion 303 is easily performed and a change in the amount of liquid to be supplied by the pump 94 can be reduced, an operation for stably supplying liquid by the liquid supply device body 16 is achieved.

Next, the operator connects the connecting pipe 127 of the tube 126 shown in Fig. 6 to the forceps port 72, and inserts the distal end opening part 129 of the tube 128 into the tank 18 through the connection port 18A of the tank 18 and positions the distal end opening part 129 below the liquid level in the tank 18. Accordingly, preparation for sending liquid, which is stored in the tank 18, to the treatment tool-insertion channel 52 is completed.

Next, the operator presses the power switch 86 to actuate the liquid supply device body 16. Accordingly, the power switch indicator lamp 87 is turned on. Further, since the cover 84 is closed in this case, the control circuit 150 shown in Fig. 10 recognizes the connection of the liquid supply tube 302 to the liquid supply device body 16 and identifies the liquid supply tube on the basis of a detection signal output from the microswitch 166. Then, the control circuit 150 reads out data, which are required to set the flow rate range of liquid to a flow rate range corresponding to the liquid supply tube 302, from the ROM 154 and outputs the data to the pump control circuit 162. Accordingly, the range of the rotational speed of the rotor 98 is set so that the amount of liquid to be supplied, which can be adjusted by the flow rate adjustment knob 88, is in the flow rate range of 400 mL/min to 700 mL/min. Further, the control circuit 150 sets the rotational speed of the rotor 98 according to the operation position of the flow rate adjustment knob 88 so that liquid is supplied at a flow rate in the range of the flow rate range. In this case, the control circuit 150 turns on the lamp 92A (see Fig. 5) of the liquid supply tube-detection indicator lamp 92 on the basis of the detection signal.

After that, in a case where the operator presses the foot switch 22, the rotor 98 is rotated at a rotational speed corresponding to the amount of liquid to be supplied (a ratio to the flow rate range) that corresponds to the operation position of the flow rate adjustment knob 88. Accordingly, liquid can be sent to the treatment tool-insertion channel 52 in a flow rate range corresponding to the liquid supply tube 302. Then, in a case where the operator wants to finely adjust the amount of liquid to be supplied, the operator operates the flow rate adjustment knob 88. As a result, the amount of liquid to be supplied can be finely adjusted without exceeding the set flow rate range. The same operation applies to a case where the tube unit 400 is fixed to the liquid supply device body 16.

After that, in a case where the operator is to detach the tube unit 300 from the liquid supply device body 16, the operator opens the cover 84, detaches the joint 104 from the liquid supply tube-detection unit 90, detaches the stopper part 108 from the restriction portion 112, and detaches the stopper part 110 from the restriction portion 114. The operator can detach the tube unit 300 from the liquid supply device body 16 merely by this work.

Even in a case where the operator is to attach each of the tube units 400 to 700 to the liquid supply device body 16 and a case where the operator is to detach each of the tube units 400 to 700 from the liquid supply device body 16, the same procedures as the procedures corresponding to the tube unit 300 are performed.

According to the liquid supply device 10 for an endoscope of the embodiment, as described above, the liquid supply tube 302 is provided with the stopper parts 108 and 110 protruding in the radial direction of the tube (the direction of the arrow C shown in Fig. 6) and the liquid supply device body 16 is provided with the restriction portions 112 and 114 that are engaged with the stopper parts 108 and 110 and restrict the positions of the stopper parts 108 and 110. Accordingly, the liquid supply tube 302 can be simply attached to and detached from the liquid supply device body 16.

Further, the liquid supply device 10 for an endoscope according to the embodiment includes the pump 94 that sends liquid by rotating the rotor 98 and squeezing each of the liquid supply tubes 302 to 702 with the rollers 100 in a state where the winding portion 303 is wound on the outer peripheral portion of the rotor 98. For this reason, the liquid supply device 10 for an endoscope according to the embodiment has the following advantage.

That is, the principle of operation of the pump 94 of the embodiment is substantially the same as that of a peristaltic pump, which is called a so-called roller pump or tube pump, in that the pump 94 includes the motor 96, the rotor 98, and the plurality of rollers 100. However, the configuration of the pump 94 is different from that of the peristaltic pump in terms of the followings.

That is, the peristaltic pump includes a semicircular wall surface around a plurality of rollers, and has well-known configuration where a winding portion is disposed in a small gap between the wall surface and the plurality of rollers in a state where the winding portion is elastically deformed (reduced in diameter) (for example, see JP1994-007372A (JP-H06-007372A)). Further, the wall surface is fixed to a pump body.

In contrast, the pump 94 of the embodiment does not include a fixed semicircular wall surface and the winding portion 303 is wound around the rotor 98 in a U shape in a case where the winding portion 303 is to be wound. According to the pump 94 of the embodiment, work requiring labor for reducing the diameter of the winding portion and disposing the winding portion in the small gap does not need to be performed unlike in the peristaltic pump. Accordingly, work for attaching the liquid supply tube 302 to the pump 94 and replacing the liquid supply tube 302 is easily performed. The pump 94 of the embodiment includes arc-shaped wall portions 95 provided below the rotor 98 as shown in Fig. 5, and each of the wall portions 95 is disposed so as to be vertically movable between a retracted position (see Fig. 5) where the wall portion is retracted from the rotor 98 in conjunction with an operation for opening the cover 84 and an advanced position (not shown) where the wall portion pinches the winding portion 303 between the roller 100 and itself in conjunction with an operation for closing the cover 84. Accordingly, in a case where the cover 84 is closed, the winding portion 303 of the liquid supply tube 302 is pinched between the roller 100 and the wall portions 95.

Further, an aspect where the joint 104 is provided with the stopper part 108 and the joint 106 is provided with the stopper part 110 has been described in the embodiment, but positions where the stopper parts 108 and 110 are provided are not limited to the configuration of the embodiment. Another example of each of the stopper parts 108 and 110 will be described below.

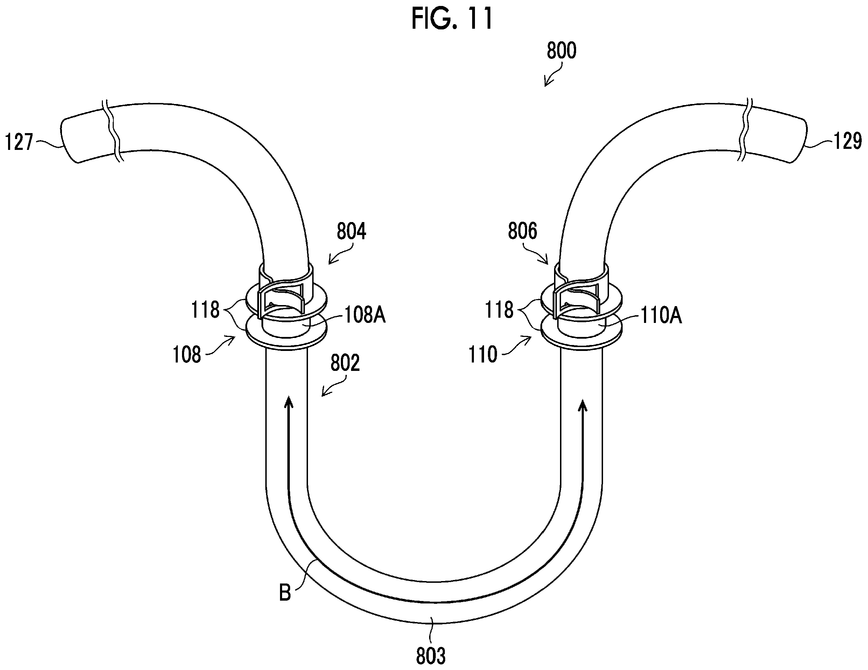

A tube unit 800 of another embodiment is shown in Fig. 11. In the description of the tube unit 800 shown in Fig. 11, the same members as the members of the tube unit 300 shown in Fig. 6 or members similar to the members of the tube unit 300 will be denoted by the same reference numerals as the reference numerals shown in Fig. 6 and the description thereof will be omitted.

The tube unit 800 shown in Fig. 11 includes one liquid supply tube 802. That is, Fig. 11 shows the tube unit 800 that does not use the joints 104 and 106 and the tubes 126 and 128 of the tube unit 300 shown in Fig. 6.

The tube unit 800 includes a winding portion 803, a stopper-forming member 804 is provided to be closer to the liquid destination side than the winding portion 803, and a stopper part 108 is formed at the stopper-forming member 804. Further, the tube unit 800 includes a stopper-forming member 806 that is provided to be closer to the liquid source side than the winding portion 803, and a stopper part 110 is formed at the stopper-forming member 806.

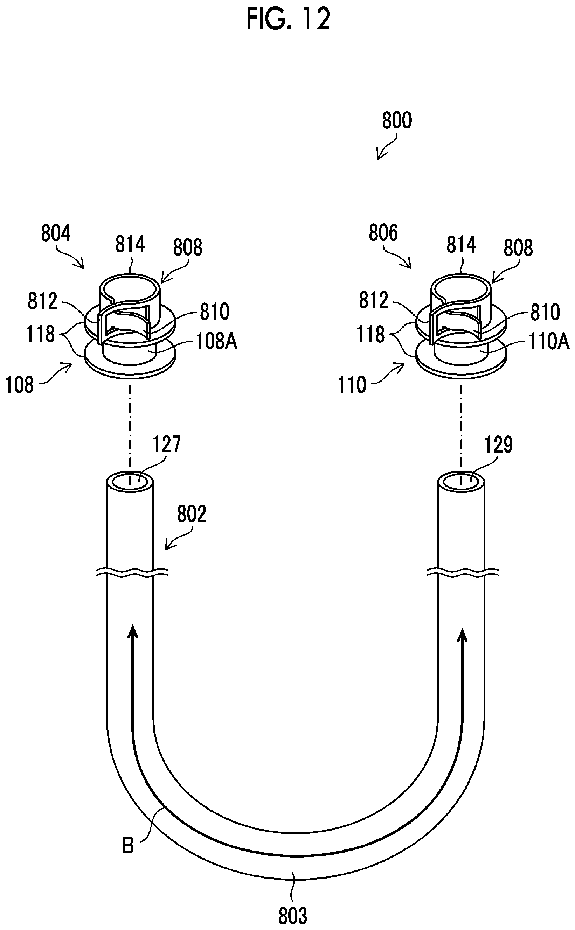

Fig. 12 is an exploded perspective view of the tube unit 800, and shows a state where the stopper-forming members 804 and 806 are not yet connected to the liquid supply tube 802.

As shown in Fig. 12, the stopper-forming member 804 includes an annular portion 808 into which the liquid supply tube 802 is to be inserted through a cylindrical shaft portion 108A of the stopper part 108 and a pair of claw portions 810 and 812 that is used to elastically increase the diameter of the annular portion 808, and the stopper-forming member 806 includes an annular portion 808 into which the liquid supply tube 802 is to be inserted through a cylindrical shaft portion 110A of the stopper part 110 and a pair of claw portions 810 and 812 that is used to elastically increase the diameter of the annular portion 808.

The annular portion 808 is formed of a belt-like leaf spring 814 that is formed in an annular shape, and is formed so that the inner diameter of the annular portion 808 is smaller than the outer diameter of the liquid supply tube 802 in a natural state shown in Fig. 12. Further, the claw portions 810 and 812 are formed to protrude from the end portions of the leaf spring 814 in a circumferential direction.

In a case where an operator is to assemble the liquid supply tube 802 and the stopper-forming members 804 and 806 into the tube unit 800, the operator picks up the claw portions 810 and 812 with fingers and causes the claw portions 810 and 812 to be close to each other to increase the diameters of the annular portions 808 and 808. After that, the operator inserts the liquid supply tube 802 into the annular portions 808 and 808 of the stopper-forming members 804 and 806 through the shaft portions 108A and 110A. Then, after positioning the stopper parts 108 and 110 in an axial direction of the tube indicated by an arrow B, the operator releases the fingers from the claw portions 810 and 812. Accordingly, since the inner diameters of the annular portions 808 and 808 become smaller than the diameter of the liquid supply tube 802, the stopper-forming members 804 and 806 are integrally connected to the liquid supply tube 802. Therefore, the tube unit 800 is assembled.

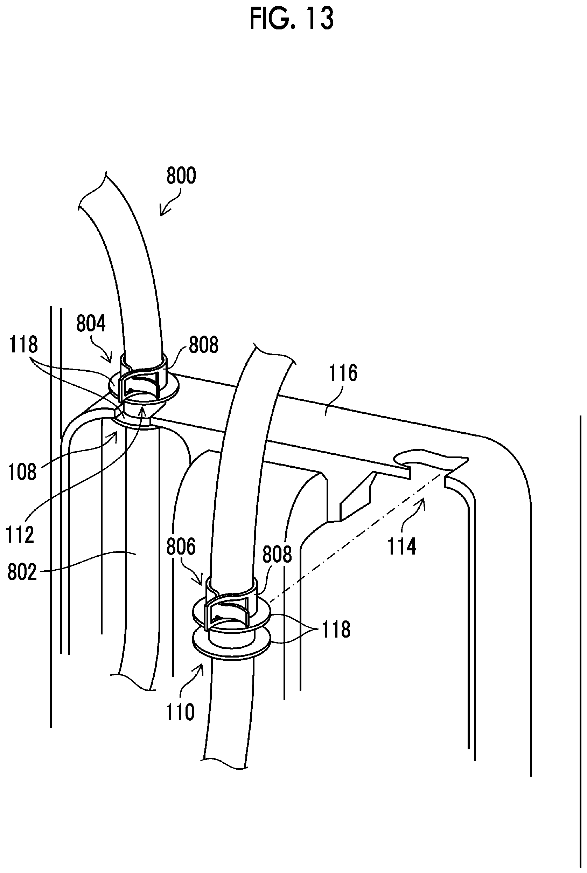

Then, in a case where the operator is to attach the tube unit 800 to the liquid supply device body 16 (see Fig. 4), the operator can attach the liquid supply tube 802 to the liquid supply device body 16 merely by engaging the stopper part 108 with the restriction portion 112 and engaging the stopper part 110 with the restriction portion 114 as shown in Fig. 13. Further, the operator can detach the liquid supply tube 802 from the liquid supply device body 16 merely by detaching the stopper part 108 from the restriction portion 112 and detaching the stopper part 110 from the restriction portion 114. Accordingly, the tube unit 800 shown in Fig. 11 can also be simply attached to and detached from the liquid supply device body 16.

Furthermore, since the positions of the stopper parts 108 and 110 in the axial direction of the liquid supply tube 802 can be adjusted in the tube unit 800, the length of the winding portion 803 to be wound on the outer peripheral portion of the rotor 98 can be adjusted. Accordingly, even in a case where the tube unit 800 is used in other liquid supply devices for an endoscope of which the lengths of the outer peripheral portions of rotors (the sizes of rotors) are different from each other, the length of the winding portion 803 can be easily adjusted to lengths corresponding to the sizes of the rotors. Therefore, the versatility of the tube unit 800 is high.

Further, one stopper-forming member of the stopper-forming members 804 and 806 may be provided with, for example, the identifier 140, 142, or 144 shown in Fig. 8. Furthermore, the operator may reduce the diameter of the liquid supply tube 802 using the elasticity of the liquid supply tube 802 without increasing the diameter of the annular portion 808 and press-fits the liquid supply tube 802 into the annular portion 808. In this case, it is preferable that the diameter of the annular portion 808 is set to be slightly smaller than the outer diameter of the liquid supply tube 802. Alternatively, the liquid supply tube 802 and the annular portion 808 may be fixed to each other by adhesion or welding.

In the embodiment, mechanical identifiers, which are formed to have different shapes by combinations of the two projection portions 146 and 148, have been exemplified as the identifiers and a mechanical reading unit typified by the microswitch 166 has been exemplified as the reading unit. However, other identifiers and other reading units may be applied.

### First example

For example, an identifier having magnetic identification information may be employed as the identifier and a reading unit, which magnetically reads the magnetic identification information, may be employed as the identifier recognition unit.

The identifier having magnetic identification information may be mounted on, for example, the liquid supply tube 302 other than the joint 104. According to an aspect where magnetic identification information is used, the degree of freedom in mounting the identifier having magnetic identification information and the reading unit is improved. For example, in a case where the identifier having magnetic identification information is mounted on the liquid supply tube 302, the identifier of the liquid supply tube 302 and the reading unit can be made close to each other as long as the reading unit is mounted in the rotor housing chamber 85. Accordingly, it is preferable that the identifier having magnetic identification information is mounted on the liquid supply tube 302.

A magnetic tape in which magnetic information indicating each of the types of the liquid supply tubes 302 to 702 is magnetically written can be exemplified as the identifier having magnetic identification information. A magnetic information reading unit including a magnetic head, which can read the magnetic information, can be exemplified as the reading unit.

### Second example

Alternatively, an identifier having electric identification information may be employed as the identifier and a reading unit, which electrically reads the electric identification information, may be employed as the identifier recognition unit.

The identifier having electric identification information may be mounted on, for example, the liquid supply tube 302 other than the joint 104. According to an aspect where electric identification information is used, the degree of freedom in mounting the identifier having electric identification information and the reading unit is improved. For example, in a case where the identifier having electric identification information is mounted on the liquid supply tube 302, the identifier of the liquid supply tube 302 and the reading unit can be made close to each other as long as the reading unit is mounted in the rotor housing chamber 85. Accordingly, it is preferable that the identifier having electric identification information is mounted on the liquid supply tube 302.

A radio frequency identifier (RFID) tag including an integrated circuit (IC) chip in which electronic information indicating each of the types of the liquid supply tubes 302 to 702 is stored can be exemplified as the identifier having electric identification information. An electric information reading unit including a reader/writer, which can read the electronic information, can be exemplified as the reading unit.

### Third example

Alternatively, an identifier having optical identification information may be employed as the identifier and a reading unit, which optically reads the optical identification information, may be employed as the identifier recognition unit.

The identifier having optical identification information may be mounted on, for example, the liquid supply tube 302 other than the joint 104. According to an aspect where optical identification information is used, the degree of freedom in mounting the identifier having optical identification information and the reading unit is improved. For example, in a case where the identifier having optical identification information is mounted on the liquid supply tube 302, the identifier of the liquid supply tube 302 and the reading unit can be made close to each other as long as the reading unit is mounted in the rotor housing chamber 85. Accordingly, it is preferable that the identifier having optical identification information is mounted on the liquid supply tube 302. Further, since the rotor housing chamber 85 is shielded from light by the cover 84, the reading unit can read optical identification information without being affected by external light.

A geometric pattern, such as a bar code, a color code, or a dot code, indicating each of the types of the liquid supply tubes 302 to 702 can be exemplified as the identifier having optical identification information; and an optical information reading unit including an image pickup element, such as a laser or a charge coupled device (CCD) capable of reading the geometric pattern, can be exemplified as the reading unit. The geometric pattern can also be mounted on each of the liquid supply tubes 302 to 702 likewise.

The invention has been described above, but it is natural that the invention is not limited to the above-mentioned examples and may have various improvements and modifications without departing from the scope of the invention.

### Explanation of References

10: liquid supply device for endoscope
12: endoscope
14: high-frequency knife
16: liquid supply device body
18: tank
18A: connection port
20: tank tray
22: foot switch
30: endoscope system
32: processor device for endoscope
34: light source device
34A: processor-side connector
35: image processing device
36: display
40: insertion unit
42: soft part
44: bendable part
46: distal end part
46A: distal end face
48: air/water supply channel
50: water jet pipe
52: treatment tool-insertion channel
52A: suction passage
52B: treatment tool-insertion passage
54: operation unit body
56: grip part
58: bending-proof pipe
60: universal cable
62: connector unit
64: electrical connector
66: liquid supply connector
68: air/water supply button
70: suction button
72: forceps port
74: liquid supply pipe line
74A: proximal end opening
76: angle knob
80: housing
82: operation panel
83: hinge portion
84: cover
84A: upper side portion
85: rotor housing chamber
86: power switch
87: power switch indicator lamp
88: flow rate adjustment knob
90: liquid supply tube-detection unit
90A: recessed portion
92: liquid supply tube-detection indicator lamp
94: pump
95: wall portion
96: motor
98: rotor
100: roller
104: joint
104A: rear surface
104B: protruding portion
106: joint
108: stopper part
108A: shaft portion
110: stopper part
110A: shaft portion
112: restriction portion
112A: groove
114: restriction portion
114A: groove
116: housing case
116A: upper side portion
118: stopper piece
120: pressing piece
126: tube
127: connecting pipe
128: tube
129: distal end opening part
130: joint body part
132: joint body part
130A: knob surface
132A: knob surface
134: knob portion
136: knob portion
140: identifier
142: identifier
144: identifier
146: projection portion
148: projection portion
150: control circuit
152: CPU
154: ROM
156: RAM
158: AC/DC power supply
160: cover opening/closing detection switch
162: pump control circuit
164: variable resistor
166: microswitch
168: actuator
170: actuator
300: tube unit
302: liquid supply tube
302A: end portion
302B: end portion
303: winding portion
400: tube unit
402: liquid supply tube
500: tube unit
502: liquid supply tube
600: tube unit
602: liquid supply tube
700: tube unit
702: liquid supply tube
800: tube unit
802: liquid supply tube
803: winding portion
804: stopper-forming member
806: stopper-forming member
808: annular portion
810: claw portion
812: claw portion
814: leaf spring

## Claims

1. A liquid supply tube configured to be detachably mounted on a liquid supply device (10) for an endoscope (12), the liquid supply device (10) including a liquid supply device body (16) comprising a motor (96) and a rotor (98) that is rotated by the motor (96), the rotor (98) including a plurality of rollers (100) provided on an outer peripheral portion thereof, wherein the liquid supply device body (16) is configured to send liquid by the motor (96) rotating the rotor (98) and the rollers (100) squeezing the liquid supply tube when the liquid supply tube is wound around the rotor (98), wherein the liquid supply device body (16) is provided with a first restriction portion (112) and a second restriction portion (114), the liquid supply tube comprising:
a winding portion (303) that is configured to be wound around the rotor (98);
a first stopper part (108) that is provided to be closer to a liquid destination side than the winding portion (303) and protrudes in a radial direction of the tube orthogonal to an axial direction of the liquid supply tube; and
a second stopper part (110) that is provided to be closer to a liquid source side than the winding portion (303) and protrudes in the radial direction of the tube,
wherein the first stopper part (108) is configured to engage the first restriction portion (112) of the liquid supply device body (16) so that a position of the first stopper part (108) is restricted, and
the second stopper part (110) is configured to engage the second restriction portion (114) of the liquid supply device body (16) so that a position of the second stopper part (110) is restricted;
wherein the first stopper part (108) includes a first shaft portion (108A) extending in the axial direction of the tube, and a first pair of stopper pieces (118) that are disposed to be spaced apart from each other in the axial direction of the tube with the first shaft portion (108A) interposed therebetween, and the first pair of stopper pieces (118) is configured to engage with the first restriction portion (112) so that movement of the first stopper part (108) to both sides in the axial direction of the tube is restricted;
wherein the second stopper part (110) includes a second shaft portion (110A) extending in the axial direction of the tube, and a second pair of stopper pieces (118) that are disposed to be spaced apart from each other in the axial direction of the tube with the second shaft portion (1110A) interposed therebetween, and the second pair of stopper pieces (118) is configured to engage with the second restriction portion (114) so that movement of the second stopper part (110) to both sides in the axial direction of the tube is restricted; and
wherein diameters of the first shaft portion (108A) and the second shaft portion (110A) are different from each other.

2. The liquid supply tube according to claim 1, further comprising:
a first j oint that is to be connected to a first tube corresponding to the liquid destination side,
wherein the first joint includes the first stopper part (108).

3. The liquid supply tube according to any one of claims 1 to 2,
wherein the liquid supply tube includes a second joint that is to be connected to a second tube corresponding to the liquid source side, and
the second joint includes the second stopper part (110).

4. The liquid supply tube according to claim 1,
wherein the liquid supply tube includes a first stopper-forming member that is to be integrally connected to the liquid supply tube, and
the first stopper part (108) is formed at the first stopper-forming member.

5. The liquid supply tube according to any one of claims 1 and 4,
wherein the liquid supply tube includes a second stopper-forming member that is to be integrally connected to the liquid supply tube, and
the second stopper part (110) is formed at the second stopper-forming member.

6. A tube unit (300) comprising:
the liquid supply tube (302) according to any one of claims 1 to 5;
a first tube (126) configured to be connected to the liquid destination side of the liquid supply tube (302) via a first joint (104),
wherein the first tube (126) includes a first connecting part (127) that is provided at an end portion thereof opposite to the first joint (104) and is capable of being connected to a liquid supply pipe line provided in an endoscope (12) or a treatment tool.

7. A tube unit (300) comprising:
the liquid supply tube (302) according to any one of claims 1 to 5;
a second tube (128) configured to be connected to the liquid destination side of the liquid supply tube (302) via a second joint (106),
wherein the second tube (128) includes a second connecting part (129) that is provided at an end portion thereof opposite to the second joint (106) and is capable of being connected to a tank in which liquid is stored.

8. A liquid supply device body (16) configured to detachably receive a liquid supply tube according to any one of claims 1 to 5, the liquid supply device body (16) comprising:
a motor (96); and
a rotor (98) that is rotated by the motor (96), wherein the rotor (98) includes a plurality of rollers (100) provided on an outer peripheral portion thereof;
wherein the liquid supply device body (16) is configured to send liquid by the motor (96) rotating the rotor (98) and the rollers (100) squeezing a liquid supply tube when the liquid supply tube is wound around the rotor (98); the liquid supply device body (16) further comprising:
a first restriction portion (112) that is configured to engage with the first stopper part (108) of the liquid supply tube so that a position of the first stopper part (108) is restricted; and
a second restriction portion (114) that is configured to engage with the second stopper part (110) of the liquid supply tube so that a position of the second stopper part (110) is restricted;
wherein the first restriction portion (112) includes a first engagement portion configured to engage the first pair of stopper pieces (118) so that movement of the first stopper part (108) to both sides in an axial direction of the liquid supply tube is restricted, and a first groove (112A) having a width corresponding to the diameter of the first shaft portion (108A); and
wherein the second restriction portion (114) includes a second engagement portion configured to engage the second pair of stopper pieces (118) so that movement of the second stopper part (110) to both sides in an axial direction of the liquid supply tube is restricted, and a second groove (114A) having a width corresponding to the diameter of the second shaft portion (110A).

9. The liquid supply device body (16) according to claim 8, further comprising:
a housing case that defines a rotor housing chamber (85) capable of housing the rotor (98),
wherein the housing case includes the first restriction portion (112) and the second restriction portion (114).

10. The liquid supply device body (16) according to claim 9,
wherein the housing case includes a cover that is capable of opening and closing the rotor housing chamber (85), and
the cover includes a pressing piece that protrudes toward any one restriction portion of the first restriction portion (112) or the second restriction portion (114), and the pressing piece presses the first stopper part (108) or the second stopper part (110) restricted by the one restriction portion and restricts movement of the first stopper part (108) or the second stopper part (110) in a radial direction of the tube orthogonal to an axial direction of the liquid supply tube.

11. A liquid supply device (10) for an endoscope (12) comprising:
a liquid supply tube according to any one of claims 1 to 5; and
a liquid supply device body (16) according to any one of claims 8 to 10.

## Patentansprüche

1. Flüssigkeitszufuhr-Rohr, das so konfiguriert ist, dass es an einer Flüssigkeitszufuhrvorrichtung (10) für ein Endoskop (12) lösbar montiert wird, wobei die Flüssigkeitszufuhrvorrichtung (10) einen Flüssigkeitszufuhrvorrichtungs-Körper (16) enthält, der einen Motor (96) und einen Rotor (98), der durch den Motor (96) gedreht wird, umfasst, wobei der Rotor (98) mehrere Rollen (100), die an einem Außenumfangsabschnitt davon vorgesehen sind, enthält, wobei der Flüssigkeitszufuhrvorrichtungs-Körper (16) so konfiguriert ist, dass er Flüssigkeit sendet, indem der Motor (96) den Rotor (98) dreht und die Rollen (100) das Flüssigkeitszufuhr-Rohr quetschen, wenn das Flüssigkeitszufuhr-Rohr um den Rotor (98) gewickelt ist, wobei der Flüssigkeitszufuhrvorrichtungs-Körper (16) mit einem ersten Begrenzungsabschnitt (112) und einem zweiten Begrenzungsabschnitt (114) versehen ist, wobei das Flüssigkeitszufuhr-Rohr umfasst:
einen Wicklungsabschnitt (303), der so konfiguriert ist, dass er um den Rotor (98) gewickelt ist;
einen ersten Anschlagteil (108), der so vorgesehen ist, dass er näher an einer Flüssigkeitszielseite als der Wicklungsabschnitt (303) liegt und in einer Radialrichtung des Rohrs senkrecht zu einer Axialrichtung des Flüssigkeitszufuhr-Rohrs vorsteht; und
einen zweiten Anschlagteil (110), der so vorgesehen ist, dass er näher an einer Flüssigkeitsquellenseite als der Wicklungsabschnitt (303) liegt und in der Radialrichtung des Rohrs vorsteht,
wobei der erste Anschlagteil (108) so konfiguriert ist, dass er den ersten Begrenzungsabschnitt (112) des Flüssigkeitszufuhrvorrichtungs-Körpers (16) so in Eingriff bringt, dass eine Position des ersten Anschlagteils (108) begrenzt wird, und
der zweite Anschlagteil (110) so konfiguriert ist, dass er den zweiten Begrenzungsabschnitt (114) des Flüssigkeitszufuhrvorrichtungs-Körpers (16) so in Eingriff bringt, dass eine Position des zweiten Anschlagteils (110) begrenzt wird;
wobei der erste Anschlagteil (108) einen ersten Schaftabschnitt (108A), der sich in der Axialrichtung des Rohrs erstreckt, und ein erstes Paar Anschlagstücke (118), die so angeordnet sind, dass sie in der Axialrichtung des Rohrs voneinander beabstandet sind, wobei der erste Schaftabschnitt (108A) dazwischen eingefügt ist, enthält und das erste Paar Anschlagstücke (118) so konfiguriert ist, dass es mit dem ersten Begrenzungsabschnitt (112) in Eingriff kommt, so dass Bewegung des ersten Anschlagteils (108) zu beiden Seiten in der Axialrichtung des Rohrs begrenzt wird;
wobei der zweite Anschlagteil (110) einen zweiten Schaftabschnitt (110A), der sich in der Axialrichtung des Rohrs erstreckt, und ein zweites Paar Anschlagstücke (118), die so angeordnet sind, dass sie in der Axialrichtung des Rohrs voneinander beabstandet sind, wobei der zweite Schaftabschnitt (1110A) dazwischen eingefügt ist, enthält und das zweite Paar Anschlagstücke (118) so konfiguriert ist, dass es mit dem zweiten Begrenzungsabschnitt (114) in Eingriff kommt, so dass Bewegung des zweiten Anschlagteils (110) zu beiden Seiten in der Axialrichtung des Rohrs begrenzt wird; und
wobei Durchmesser des ersten Schaftabschnitts (108A) und des zweiten Schaftabschnitts (110A) voneinander unterschiedlich sind.

2. Flüssigkeitszufuhr-Rohr nach Anspruch 1, ferner umfassend:
eine erste Verbindung, die mit einem ersten Rohr, das der Flüssigkeitszielseite entspricht, zu verbinden ist,
wobei die erste Verbindung den ersten Anschlagteil (108) enthält.

3. Flüssigkeitszufuhr-Rohr nach einem der Ansprüche 1 bis 2,
wobei das Flüssigkeitszufuhr-Rohr eine zweite Verbindung enthält, die mit einem zweiten Rohr, das der Flüssigkeitsquellenseite entspricht, zu verbinden ist, und
wobei die zweite Verbindung den zweiten Anschlagteil (110) enthält.

4. Flüssigkeitszufuhr-Rohr nach Anspruch 1,
wobei das Flüssigkeitszufuhr-Rohr ein erstes Anschlagformungselement enthält, das integral mit dem Flüssigkeitszufuhr-Rohr zu verbinden ist, und
der erste Anschlagteil (108) an dem ersten Anschlagformungselement gebildet ist.

5. Flüssigkeitszufuhr-Rohr nach einem der Ansprüche 1 und 4,
wobei das Flüssigkeitszufuhr-Rohr ein zweites Anschlagformungselement enthält, das integral mit dem Flüssigkeitszufuhr-Rohr zu verbinden ist, und
der zweite Anschlagteil (110) an dem zweiten Anschlagformungselement gebildet ist.

6. Rohreinheit (300), umfassend:
das Flüssigkeitszufuhr-Rohr (302) nach einem der Ansprüche 1 bis 5;
ein erstes Rohr (126), das so konfiguriert ist, via eine erste Verbindung (104) mit der Flüssigkeitszielseite des Flüssigkeitszufuhr-Rohrs (302) verbunden zu werden,
wobei das erste Rohr (126) einen ersten Verbindungsabschnitt (127) enthält, der an einem Endabschnitt davon, der der ersten Verbindung (104) gegenüberliegt,
vorgesehen ist und mit einer Flüssigkeitszufuhr-Rohrleitung, die bei einem Endoskop (12) oder einem Behandlungswerkzeug vorgesehen ist, verbunden werden kann.

7. Rohreinheit (300), umfassend:
das Flüssigkeitszufuhr-Rohr (302) nach einem der Ansprüche 1 bis 5;
ein zweites Rohr (128), das so konfiguriert ist, via eine zweite Verbindung (106) mit der Flüssigkeitszielseite des Flüssigkeitszufuhr-Rohrs (302) verbunden zu werden,
wobei das zweite Rohr (128) einen zweiten Verbindungsabschnitt (129) enthält, der an einem Endabschnitt davon, der der zweiten Verbindung (106) gegenüberliegt,
vorgesehen ist und mit einem Tank, in dem Flüssigkeit gespeichert wird, verbunden werden kann.

8. Flüssigkeitszufuhrvorrichtungs-Körper (16), der so konfiguriert ist, dass er ein Flüssigkeitszufuhr-Rohr nach einem der Ansprüche 1 bis 5 lösbar aufnimmt, wobei der Flüssigkeitszufuhrvorrichtungs-Körper (16) umfasst:
einen Motor (96); und
einen Rotor (98), der durch den Motor (96) gedreht wird, wobei der Rotor (98) mehrere Rollen (100), die an einem Außenumfangsabschnitt davon vorgesehen sind, enthält;
wobei der Flüssigkeitszufuhrvorrichtungs-Körper (16) so konfiguriert ist, dass er Flüssigkeit sendet, indem der Motor (96) den Rotor (98) dreht und die Rollen (100) ein Flüssigkeitszufuhr-Rohr quetschen, wenn das Flüssigkeitszufuhr-Rohr um den Rotor (98) gewickelt ist; wobei der Flüssigkeitszufuhrvorrichtungs-Körper (16) ferner umfasst:
einen ersten Begrenzungsabschnitt (112), der so konfiguriert ist, dass er mit dem ersten Anschlagteil (108) des Flüssigkeitszufuhr-Rohrs in Eingriff kommt, so dass eine Position des ersten Anschlagteils (108) begrenzt wird; und
einen zweiten Begrenzungsabschnitt (114), der so konfiguriert ist, dass er mit dem zweiten Anschlagteil (110) des Flüssigkeitszufuhr-Rohrs in Eingriff kommt, so dass eine Position des zweiten Anschlagteils (110) begrenzt wird;
wobei der erste Begrenzungsabschnitt (112) einen ersten Eingriffsabschnitt, der so konfiguriert ist, dass er das erste Paar Anschlagstücke (118) in Eingriff bringt, so dass Bewegung des ersten Anschlagteils (108) zu beiden Seiten in einer Axialrichtung des Flüssigkeitszufuhr-Rohrs begrenzt wird, und eine erste Nut (112A), die eine Breite aufweist, die dem Durchmesser des ersten Schaftabschnitts (108A) entspricht, enthält; und
wobei der zweite Begrenzungsabschnitt (114) einen zweiten Eingriffsabschnitt, der so konfiguriert ist, dass er das zweite Paar Anschlagstücke (118) in Eingriff bringt, so dass Bewegung des zweiten Anschlagteils (110) zu beiden Seiten in einer Axialrichtung des Flüssigkeitszufuhr-Rohrs begrenzt wird, und eine zweite Nut (114A), die eine Breite aufweist, die dem Durchmesser des zweiten Schaftabschnitts (110A) entspricht, enthält.

9. Flüssigkeitszufuhrvorrichtungs-Körper (16) nach Anspruch 8, ferner umfassend:
einen Gehäusekasten, der eine Rotorgehäusekammer (85), die in der Lage ist, den Rotor (98) aufzunehmen, definiert,
wobei der Gehäusekasten den ersten Begrenzungsabschnitt (112) und den zweiten Begrenzungsabschnitt (114) enthält.

10. Flüssigkeitszufuhrvorrichtungs-Körper nach Anspruch 9,
wobei der Gehäusekasten eine Abdeckung enthält, die in der Lage ist, die Rotorgehäusekammer (85) zu öffnen und zu schließen, und
die Abdeckung ein Druckstück enthält, das zu einem beliebigen Begrenzungsabschnitt von dem ersten Begrenzungsabschnitt (112) oder dem zweiten Begrenzungsabschnitt (114) hin vorsteht, und das Druckstück den ersten Anschlagteil (108) oder den zweiten Anschlagteil (110), der durch den einen Begrenzungsabschnitt begrenzt ist, drückt und Bewegung des ersten Anschlagteils (108) oder des zweiten Anschlagteils (110) in einer Radialrichtung des Rohrs senkrecht zu einer Axialrichtung des Flüssigkeitszufuhr-Rohrs begrenzt.

11. Flüssigkeitszufuhrvorrichtung (10) für ein Endoskop (12), umfassend:
ein Flüssigkeitszufuhr-Rohr nach einem der Ansprüche 1 bis 5; und
einen Flüssigkeitszufuhrvorrichtungs-Körper (16) nach einem der Ansprüche 8 bis 10.

## Revendications

1. Tube d'alimentation en liquide configuré pour être monté de manière amovible sur un dispositif d'alimentation en liquide (10) pour un endoscope (12), le dispositif d'alimentation en liquide (10) incluant un corps de dispositif d'alimentation en liquide (16) comprenant un moteur (96) et un rotor (98) qui est mis en rotation par le moteur (96), le rotor (98) incluant une pluralité de rouleaux (100) prévus sur une partie périphérique extérieure de celui-ci, dans lequel le corps de dispositif d'alimentation en liquide (16) est configuré pour acheminer un liquide par la rotation du rotor (98) par le moteur (96) et par le pincement du tube d'alimentation en liquide par les rouleaux (100) lorsque le tube d'alimentation en liquide est enroulé autour du rotor (98), dans lequel le corps de dispositif d'alimentation en liquide (16) est pourvu d'une première partie de restriction (112) et d'une deuxième partie de restriction (114), le tube d'alimentation en liquide comprenant :
une partie d'enroulement (303) qui est configurée pour être enroulée autour du rotor (98) ;
une première partie de butée (108) qui est prévue pour être plus proche d'un côté de destination de liquide que la partie d'enroulement (303) et qui fait saillie dans une direction radiale du tube orthogonale à une direction axiale du tube d'alimentation en liquide ; et
une deuxième partie de butée (110) qui est prévue pour être plus proche d'un côté de source de liquide que la partie d'enroulement (303) et qui fait saillie dans la direction radiale du tube,
dans lequel la première partie de butée (108) est configurée pour s'engager avec la première partie de restriction (112) du corps de dispositif d'alimentation en liquide (16) de sorte qu'une position de la première partie de butée (108) soit restreinte, et
la deuxième partie de butée (110) est configurée pour s'engager avec la deuxième partie de restriction (114) du corps de dispositif d'alimentation en liquide (16) de sorte qu'une position de la deuxième partie de butée (110) soit restreinte ;
dans lequel la première partie de butée (108) inclut une première partie d'arbre (108A) s'étendant dans la direction axiale du tube, et une première paire de pièces de butée (118) qui sont disposées de manière à être espacées l'une de l'autre dans la direction axiale du tube avec la première partie d'arbre (108A) interposée entre elles, et la première paire de pièces de butée (118) est configurée pour s'engager avec la première partie de restriction (112) de sorte que le mouvement de la première partie de butée (108) vers les deux côtés dans la direction axiale du tube soit restreint ;
dans lequel la deuxième partie de butée (110) inclut une deuxième partie d'arbre (110A) s'étendant dans la direction axiale du tube, et une deuxième paire de pièces de butée (118) qui sont disposées de manière à être espacées l'une de l'autre dans la direction axiale du tube avec la deuxième partie d'arbre (1110A) interposée entre elles, et la deuxième paire de pièces de butée (118) est configurée pour s'engager avec la deuxième partie de restriction (114) de sorte que le mouvement de la deuxième partie de butée (110) vers les deux côtés dans la direction axiale du tube soit restreint ; et
dans lequel des diamètres de la première partie d'arbre (108A) et de la deuxième partie d'arbre (110A) sont différents l'un de l'autre.

2. Tube d'alimentation en liquide selon la revendication 1, comprenant en outre :
un premier raccord qui doit être connecté à un premier tube correspondant au côté de destination de liquide,
dans lequel le premier raccord inclut la première partie de butée (108).

3. Tube d'alimentation en liquide selon l'une quelconque des revendications 1 à 2,
dans lequel le tube d'alimentation en liquide inclut un deuxième raccord qui doit être connecté à un deuxième tube correspondant au côté de source de liquide, et
dans lequel le deuxième raccord inclut la deuxième partie de butée (110).

4. Tube d'alimentation en liquide selon la revendication 1,
dans lequel le tube d'alimentation en liquide inclut un premier élément de formation de butée qui doit être connecté de manière intégrale au tube d'alimentation en liquide, et
la première partie de butée (108) est formée sur le premier élément de formation de butée.

5. Tube d'alimentation en liquide selon l'une quelconque des revendications 1 et 4,
dans lequel le tube d'alimentation en liquide inclut un deuxième élément de formation de butée qui doit être connecté de manière intégrale au tube d'alimentation en liquide, et
dans lequel la deuxième partie de butée (110) est formée sur le deuxième élément de formation de butée.

6. Unité de tube (300) comprenant :
le tube d'alimentation en liquide (302) selon l'une quelconque des revendications 1 à 5 ;
un premier tube (126) configuré pour être connecté au côté de destination de liquide du tube d'alimentation en liquide (302) via un premier raccord (104),
dans lequel le premier tube (126) inclut une première partie de connexion (127) qui est prévue à une partie d'extrémité de celui-ci opposée au premier raccord (104) et qui est capable d'être connectée à une conduite d'alimentation en liquide prévue dans un endoscope (12) ou un outil de traitement.

7. Unité de tube (300) comprenant :
le tube d'alimentation en liquide (302) selon l'une quelconque des revendications 1 à 5 ;
un deuxième tube (128) configuré pour être connecté au côté de destination de liquide du tube d'alimentation en liquide (302) via un deuxième raccord (106),
dans lequel le deuxième tube (128) inclut une deuxième partie de connexion (129) qui est prévue à une partie d'extrémité de celui-ci opposée au deuxième raccord (106) et qui est capable d'être connectée à un réservoir dans lequel un liquide est stocké.

8. Corps de dispositif d'alimentation en liquide (16) configuré pour recevoir de manière amovible un tube d'alimentation en liquide selon l'une quelconque des revendications 1 à 5, le corps de dispositif d'alimentation en liquide (16) comprenant :
un moteur (96) ; et
un rotor (98) qui est mis en rotation par le moteur (96), dans lequel le rotor (98) inclut une pluralité de rouleaux (100) prévus sur une partie périphérique extérieure de celui-ci ;
dans lequel le corps de dispositif d'alimentation en liquide (16) est configuré pour acheminer un liquide par la rotation du rotor (98) par le moteur (96) et par le pincement d'un tube d'alimentation en liquide par les rouleaux (100) lorsque le tube d'alimentation en liquide est enroulé autour du rotor (98) ; le corps de dispositif d'alimentation en liquide (16) comprenant en outre :
une première partie de restriction (112) qui est configurée pour s'engager avec la première partie de butée (108) du tube d'alimentation en liquide de sorte qu'une position de la première partie de butée (108) soit restreinte ; et
une deuxième partie de restriction (114) qui est configurée pour s'engager avec la deuxième partie de butée (110) du tube d'alimentation en liquide de sorte qu'une position de la deuxième partie de butée (110) soit restreinte ;
dans lequel la première partie de restriction (112) inclut une première partie d'engagement configurée pour s'engager avec la première paire de pièces de butée (118) de sorte que le mouvement de la première partie de butée (108) vers les deux côtés dans une direction axiale du tube d'alimentation en liquide soit restreint, et une première rainure (112A) ayant une largeur correspondant au diamètre de la première partie d'arbre (108A) ; et
dans lequel la deuxième partie de restriction (114) inclut une deuxième partie d'engagement configurée pour engager la deuxième paire de pièces de butée (118) de sorte que le mouvement de la deuxième partie de butée (110) vers les deux côtés dans une direction axiale du tube d'alimentation en liquide est restreint, et une deuxième rainure (114A) ayant une largeur correspondant au diamètre de la deuxième partie d'arbre (110A).

9. Corps de dispositif d'alimentation en liquide (16) selon la revendication 8, comprenant en outre :
un boîtier de logement qui définit une chambre de logement de rotor (85) capable de loger le rotor (98),
dans lequel le boîtier de logement inclut la première partie de restriction (112) et la deuxième partie de restriction (114).

10. Corps de dispositif d'alimentation en liquide (16) selon la revendication 9,
dans lequel le boîtier de logement inclut un couvercle qui est capable d'ouvrir et de fermer la chambre de logement de rotor (85), et
le couvercle inclut une pièce de pression qui fait saillie vers l'une quelconque des parties de restriction de la première partie de restriction (112) ou de la deuxième partie de restriction (114), et la pièce de pression presse la première partie de butée (108) ou la deuxième partie de butée (110) restreinte par l'une partie de restriction et restreint le mouvement de la première partie de butée (108) ou de la deuxième partie de butée (110) dans une direction radiale du tube orthogonale à une direction axiale du tube d'alimentation en liquide.

11. Dispositif d'alimentation en liquide (10) pour un endoscope (12) comprenant :
un tube d'alimentation en liquide selon l'une quelconque des revendications 1 à 5 ; et
un corps de dispositif d'alimentation en liquide (16) selon l'une quelconque des revendications 8 à 10.
